# FASCICULE DE BREVET EUROPEEN

(11) **EP 3 216 479 B1**
(45) Date de publication et mention de la délivrance du brevet: **17.04.2019**
(21) Numéro de dépôt: 17154965.2
(22) Date de dépôt: 07.02.2017
(51) Int. Cl.: A61M 16/10, A61H 31/00, G09B 23/28, A61M 16/00

(54) **APPAREIL D`ASSISTANCE RESPIRATOIRE**
VORRICHTUNG ZUR ATMUNGSUNTERSTÜTZUNG
RESPIRATORY ASSISTANCE APPARATUS

(30) Priorité: 07.03.2016 FR 1651885
(43) Date de publication de la demande: 13.09.2017
(73) Titulaire: Air Liquide Medical Systems, 92160 Antony (FR)
(72) Inventeur: FOURNIER, Maxence, 75015 Paris (FR); JACQUOT, Eric, 92160 Antony (FR); LIBARDI, Mickaël, 94270 Le Kremlin-Bicêtre (FR)
(74) Mandataire: Pittis, Olivier

(56) Documents cités:
- EP-A1- 2 198 823
- EP-A2- 2 343 097
- FR-A1- 3 000 893
- US-A- 6 155 257
- US-A1- 2002 069 878
- US-A1- 2010 036 266

## Description

L'invention concerne un dispositif ou appareil de ventilation artificielle, aussi appelé appareil d'assistance respiratoire ou ventilatoire, et plus particulièrement un appareil d'assistance respiratoire utilisable par un secouriste, tel un médecin du SAMU, un pompier, un infirmier ou analogue, lorsque celui-ci pratique un massage cardiaque sur une personne, c'est-à-dire un patient, en arrêt cardio-pulmonaire, pour ventiler ladite personne pendant qu'elle est soumise à des compressions thoraciques.

Certains types de ventilateurs ou appareils d'assistance respiratoire équipent les véhicules de premiers secours qui interviennent notamment sur les accidents de la circulation ou en cas d'urgence vitale, telles les crises cardiaques.

Ainsi, en cas de crise cardiaque, il est primordial d'associer une ventilation mécanique au massage cardiaque pratiqué sur la personne en arrêt cardio-pulmonaire, c'est-à-dire de pratiquer une réanimation cardiopulmoniare (RCP).

De nombreuses études révèlent que la fréquence des massages cardiaques est un caractère déterminant de son efficacité. Un consensus existe pour fixer la fréquence minimale du massage cardiaque à 100 compressions par minute. Cette fréquence minimale est notamment préconisée par : American Heart Association dans « Guidelines for Cardiopulmonary Resuscitation (CPR) and Emergency Cardiovascular Care (ECC) »; 2010*.*

Or, en pratique, il a été constaté que les massages cardiaques pratiqués sont souvent trop lents, comme en atteste : « Chest Compression Rates During Cardiopulmonary Resuscitation Are Suboptimal : A Prospective Study During In-Hospital Cardiac Arrest » ; American Heart Association ; Abella et Al, 2005

Dès lors, il apparaît comme indispensable de fournir au secouriste ou analogue, une information relative à la fréquence du massage cardiaque qu'il effectue de manière à lui permettre d'ajuster son massage cardiaque, c'est-à-dire d'augmenter les compressions thoraciques (CT) si elles sont insuffisantes pour atteindre la fréquence minimale préconisée.

Le document US-A-5496257 décrit un dispositif à placer sur le thorax du patient, durant le massage cardiaque, qui est capable de mesurer la fréquence des CT et de fournir des informations relatives à cette fréquence, et le document US-A-6390996 décrit un dispositif analogue qui se fixe autour du poignet du secouriste.

Un inconvénient majeur de tels dispositifs est d'ajouter des dispositifs supplémentaires à l'ensemble des équipements nécessaires au secouriste, ralentissant ainsi la mise en place du massage cardiaque lors de la RCP, alors que la rapidité est déterminante dans la survie du patient.

De plus, le premier dispositif rend l'intervention plus complexe car il doit être positionné entre les mains du secouriste et le thorax du patient, alors que le second dispositif cité ne permet pas à deux secouristes de se relayer aisément durant le massage, sauf si chacun est pourvu d'un tel dispositif.

Un problème majeur est donc de pouvoir fournir des informations de fréquence de CT au secouriste, sans avoir recours à un dispositif supplémentaire afin de ne pas complexifier la tâche du secouriste.

Pour tenter d'y remédier, le document FR-A-3000893 enseigne un appareil d'assistance respiratoire comprenant une source de gaz, telle une turbine motorisée, pour délivrer, par intermittence, un gaz respiratoire dans un circuit ventilatoire alimentant un patient subissant une RCP ; des moyens de mesure aptes à mesurer et à délivrer un signal représentatif d'un paramètre représentatif du flux de gaz, tel que débit, pression... ; des moyens de traitement de signal pour analyser ledit signal et en déduire la réalisation ou non d'un massage cardiaque, c'est-à-dire l'existence ou l'absence de CT, et à en déterminer la fréquence ; et un écran d'affichage pour afficher la fréquence des CT.

Or, en pratique, il a été constaté qu'avec ce type de dispositif, l'existence des CT n'est pas toujours correctement détectée pendant les intervalles de temps pendant lesquels le gaz est insufflé au patient, ce qui fausse le calcul de la fréquence desdites CT et conduit à délivrer des informations erronées au secouriste ou autre personnel soignant.

Le document EP 2 198 823 A1 enseigne un appareil de ventilation avec mode RCP (i.e. réanimation cardio pulmonaire). Cet appareil permet de comptabiliser les compressions thoraciques (CTs) à partir des flux de gaz générés par ces CTs et d'en déduire la fréquence des CTs. Une interface graphique (GUI) permet d'afficher la valeur de la fréquence qui est déterminée pendant les CTs. En d'autres termes, le ventilateur affiche la fréquence des CTs, lorsque des CTs sont détectées.

Le problème qui se pose est dès lors de proposer un appareil d'assistance respiratoire permettant de résoudre tout ou partie des problèmes et inconvénients susmentionnés, en particulier un appareil d'assistance respiratoire permettant de fournir des informations fiables de fréquence de CT au secouriste, y compris lorsque l'existence de telles CT n'est pas correctement détectée par l'appareil et ce, sans avoir recours à un dispositif supplémentaire afin de ne pas complexifier la tâche du secouriste.

La solution est alors un appareil d'assistance respiratoire comprenant :
- une source de gaz apte à délivrer un flux de gaz respiratoire dans un circuit ventilatoire destiné à être relié à un patient, le flux de gaz respiratoire étant délivré par ladite source de gaz (4) de manière à générer et à maintenir plusieurs niveaux de pression de gaz (Ph, Pb), pendant des durées (Dh, Db) préfixées,
- des moyens de mesure aptes à et conçus pour mesurer au moins un paramètre de flux représentatif du flux de gaz choisi parmi la pression du gaz et le débit de gaz, et pour délivrer au moins un signal de flux représentatif dudit paramètre de flux,
- des moyens de traitement de signal conçus pour et aptes à, c'est-à-dire configurés pour :
   i) analyser et déduire dudit signal de flux, une existence ou une absence de compressions thoraciques pratiquées sur ledit patient,
   ii) déterminer la valeur de fréquence (F) desdites compressions thoraciques en cas d'existence de compressions thoraciques,
   iii) fournir ladite valeur de fréquence (F) à des moyens d'affichage,
- et des moyens d'affichage permettant d'afficher des informations utiles au secouriste, en particulier la valeur de fréquence (F) des compressions thoraciques ou toute autre donnée ou information utile,
caractérisé en ce que les moyens de traitement de signal sont adaptés pour fournir aux moyens d'affichage, une valeur de fréquence corrigée (F_{c}) correspondant à la valeur de fréquence (F) déterminée immédiatement avant le début d'une durée (Dh, Db) considérée, lorsque les moyens de traitement de signal ne détectent pas de compression thoracique pendant une partie de ladite durée (Dh, Db) considérée.

En d'autres termes, la source de gaz est configurée pour délivrer un flux de gaz respiratoire à plusieurs niveaux de pression de gaz (Ph, Pb), pendant des durées (Dh, Db) préfixées, et l'appareil de l'invention a un fonctionnement basé sur la prise en compte de compressions thoraciques (CT) virtuelles, c'est-à-dire de CT non détectées pendant les durées (Dh, Db) de maintien de niveaux de pression de gaz (Ph, Pb) alors qu'elles ont bien lieu, et sur un affichage d'une estimation de la fréquence de ces CT non détectées. En effet, lorsque la ventilation mécanique induit des variations importantes des signaux de pression et de débit, les CT réelles peuvent être masquées et la fréquence calculée peut être erronée.

Selon l'invention, des CT virtuelles sont alors ajoutées si aucune CT réelle n'est détectée, de manière à compenser la perte d'information due aux interférences entre le massage cardiaque et la ventilation mécanique, c'est-à-dire la fourniture de gaz par la source de gaz et le maintien de niveaux de pression de gaz, typiquement une micro-soufflante motorisée, équipant l'appareil de ventilation.

Dit autrement, une fréquence de CT corrigée (F_{c}) est obtenue par estimation de CT virtuelles, ce qui revient à prendre en compte et à afficher non pas une fréquence (F) réelle qui serait erronée, mais une fréquence corrigée (F_{c}) ou virtuelle correspondant à la fréquence qui a été déterminée par l'appareil, et préférentiellement mémorisée, juste avant le début de chaque durée pendant laquelle un niveau de pression de gaz est maintenu.

Grâce à l'invention, le calcul et l'affichage de la fréquence corrigée des CT à l'aide d'estimations de CT virtuelles en cas de non-détection de massage cardiaque par l'appareil, alors qu'un massage a lieu, permettent de guider au mieux le secouriste effectuant le massage cardiaque sur patient ventilé et lui permet de s'assurer que la fréquence minimale de 100 compressions par minute est bien respectée ou, le cas échéant, de corriger sa manière d'appliquer le massage pour obtenir cette fréquence minimale.

Selon le cas, l'appareil de l'invention peut comprendre l'une ou plusieurs des caractéristiques techniques suivantes :
- les moyens d'affichage sont adaptés pour afficher ladite valeur de fréquence corrigée (F_{c}), pendant au moins une partie de la durée (Dh, Db) considérée.
- les moyens d'affichage sont conçus et/ou configurés pour afficher directement ou indirectement la fréquence. Ainsi, ils peuvent afficher directement une (ou des) valeur de fréquence, ou indirectement via une représentation graphique de valeur fréquence, par exemple sous forme d'un barre-graphe ou similaire, ou via une (des) indication de type « trop rapide », « trop lent », « accélérer le massage », « ralentir le massage », ou tout autre symbole illustratif adéquat... Ceci fournit au secouriste des informations très utiles quant à l'efficacité du massage cardiaque qu'il est en train de pratiquer.

- les moyens d'affichage sont conçus et/ou configurés pour afficher, directement ou indirectement, des informations autres que celles liées à la fréquence.
- les moyens d'affichage comprennent un écran d'affichage ou analogue, c'est-à-dire un afficheur ou un écran digital, de préférence tactile.
- la source de gaz délivre le flux de gaz respiratoire et maintient les niveaux de pression de gaz (Ph, Pb) dudit flux, pendant des durées préfixées (Dh, Db), de sorte que :
   a) le gaz est maintenu à pression haute (Ph) pendant une première durée (Dh), et
   b) le gaz est maintenu à pression basse (Pb) pendant une deuxième durée (Db), avec 0 < Pb < Ph, la deuxième durée (Db) succédant à la première durée (Dh).
- la source de gaz maintient les niveaux de pression de gaz (Ph, Pb) dudit flux, pendant des durées préfixées (Dh, Db) alternées de façon cyclique.
- la première durée (Dh) de la première phase est inférieure à 4 secondes.
- la deuxième durée (Db) de la deuxième phase (Db) est inférieure à 12 secondes.
- la durée totale de chaque cycle (Dh + Db) est inférieure à 15 secondes, typiquement inférieure à 8 secondes, typiquement de l'ordre de 5 secondes.
- la pression haute (Ph) délivrée par la source de gaz est comprise entre 100 et 400 mm H₂O, de préférence entre 150 et 300 mm H₂O, typiquement de l'ordre de 200 mm H₂O.
- la pression basse (Pb) délivrée par la source de gaz est comprise entre 20 et 90 mm H₂O, de préférence entre 30 et 70 mm H₂O, typiquement de l'ordre de 40 mm H₂O.
- il comprend des moyens de commande pilotant la source de gaz de manière à délivrer le flux de gaz et à générer les niveaux de pression (Ph, Pb) pendant les durées préfixées (Dh, Db).
- les moyens de commande pilotent la source de gaz de manière à délivrer le flux de gaz à pression haute (Ph) et/ou à pression basse (Pb).
- la source de gaz est une micro-soufflante motorisée.
- les moyens de mesure comprennent un capteur de débit ou un capteur de pression.
- les moyens de traitement de signal sont configurés pour fournir aux moyens d'affichage, la valeur de fréquence corrigée (F_{c}), lorsque les moyens de traitement de signal ne détectent pas de compression thoracique pendant une durée égale à 10 à 60% de la durée (Dh, Db) considérée.
- les moyens de traitement de signal comprennent au moins un microprocesseur mettant en oeuvre au moins un algorithme.
- il comporte des moyens de filtrage configurés pour filtrer le signal de flux représentatif du paramètre de flux provenant des moyens de mesure et fournir un signal de flux filtré aux moyens de traitement de signal.
- les moyens de traitement de signal sont configurés pour analyser un signal de flux filtré et en déduire une existence ou une absence de compressions thoraciques (CT) pratiquées sur un patient.
- les moyens de mesure permettent de mesurer au moins un paramètre de flux au sein dudit circuit ventilatoire.
- les moyens de traitement de signal sont adaptés, notamment programmés, pour comparer ledit au moins un signal de flux à au moins une valeur seuil représentative de la réalisation d'un massage cardiaque, en particulier une valeur seuil préfixée et/ou mémorisée par l'appareil, en particulier dans des moyens de mémorisation d'informations.
- il comporte en outre des moyens de mesure de durée permettant, c'est-à-dire aptes à et conçus pour, déterminer la durée pendant laquelle le massage cardiaque est réalisé.
- les moyens de mesure de durée comprennent un chronomètre intégré à un processeur.
- les moyens d'affichage sont adaptés pour afficher en outre la durée de massage cardiaque.
- il comporte des moyens de mémorisation conçus pour et aptes à mémoriser des données, de préférence une (ou plusieurs) carte mémoire, par exemple une mémoire flash.
- les moyens de traitement de signal sont configurés pour déterminer la valeur de fréquence (F) des compressions thoraciques en calculant sur une durée donnée, le rapport entre le nombre de compressions thoraciques et ladite durée donnée ; ledit rapport obtenu correspond alors à la fréquence F des CT. De préférence, la durée est comprise entre 1 et 15 secondes, de préférence entre 2 et 10 secondes, typiquement de l'ordre de 4 à 8 secondes, par exemple 6 secondes environ.
- il comporte des moyens de comptabilisation des compressions thoraciques CT détectées pendant une période de temps donnée, par exemple les moyens de comptabilisation comprennent un compteur de CT réinitialisé au début de ladite période de temps, et incrémenté lors de la détection de chaque nouvelle CT au cours de ladite période de temps.
- les moyens de comptabilisation coopèrent avec les moyens de mémorisation en fournissant auxdits moyens de mémorisation, un nombre de CT détectées sur une période donnée.
- il comporte des moyens d'alimentation en courant électrique, de préférence une ou plusieurs batteries, de préférence au moins une batterie rechargeable. Les moyens d'alimentation en courant électrique alimentent les différents composants de l'appareil, notamment l'écran d'affichage, les moyens de traitement d'information, les moyens de mesure, etc...
- il comporte un moyen de mise en route et/ou d'arrêt du fonctionnement de l'appareil, par exemple une touche, un bouton ou analogue de type marche/arrêt (on/off).

Selon un autre aspect, l'invention concerne aussi une méthode de ventilation d'une personne, c'est-à-dire un patient, en arrêt cardio-pulmonaire, comprenant les étapes de :
i) pratiquer un massage cardiaque sur une personne en arrêt cardio-pulmonaire, en opérant des compressions thoraciques sur ladite personne, et
ii) ventiler la personne en arrêt cardio-pulmonaire, de préférence simultanément à l'étape i), en administrant à ladite personne un flux de gaz respiratoire issu d'un appareil d'assistance respiratoire selon la présente invention, le flux de gaz respiratoire étant délivré par la source de gaz (4) de l'appareil d'assistance respiratoire selon la présente invention de manière à générer et à maintenir plusieurs niveaux de pression de gaz (Ph, Pb), pendant des durées (Dh, Db) préfixées,
iii) mesurer au moyen de l'appareil d'assistance respiratoire selon la présente invention au moins un paramètre de flux représentatif du flux de gaz choisi parmi la pression du gaz et le débit de gaz, et pour délivrer au moins un signal de flux représentatif dudit paramètre de flux,
iv) traiter ledit signal de flux au sein de l'appareil d'assistance respiratoire selon la présente invention de manière à en déduire, une existence ou une absence de compressions thoraciques pratiquées sur ledit patient, et déterminer la valeur de fréquence (F) desdites compressions thoraciques en cas d'existence de compressions thoraciques, et
v) afficher, de préférence sur des moyens d'affichage, tel un écran de visualisation ou analogue, de l'appareil d'assistance respiratoire selon la présente invention, une valeur de fréquence corrigée (F_{c}) correspondant à la valeur de fréquence (F) déterminée immédiatement avant le début d'une durée (Dh, Db) considérée, lorsque les moyens de traitement de signal ne détectent pas de compression thoracique pendant une partie de ladite durée (Dh, Db) considérée.

Selon le cas, on peut afficher directement ou indirectement la fréquence. Ainsi, on peut afficher directement une (ou des) valeur de fréquence, ou indirectement via une représentation graphique de valeur fréquence, par exemple sous forme d'un barre-graphe ou similaire, ou via une (des) indication de type « trop rapide », « trop lent », « accélérer le massage », « ralentir le massage », ou tout autre symbole illustratif adéquat... Ceci fournit au secouriste des informations très utiles quant à l'efficacité du massage cardiaque qu'il est en train de pratiquer.

La méthode de l'invention peut en outre comprendre tout ou partie des autres caractéristiques décrites dans la présente description.

La présente invention va maintenant être décrite plus en détail en référence aux Figures annexées parmi lesquelles :
- la Figure 1 représente un mode de réalisation d'un appareil d'assistance respiratoire selon la présente invention ;
- la Figure 2 est un exemple d'un enregistrement du paramètre de pression d'un appareil d'assistance respiratoire en l'absence de massage cardiaque ;
- la Figure 3 illustre le fonctionnement d'un exemple d'un algorithme mis en oeuvre par les moyens de commande du ventilateur de la Figure 1 selon la présente invention.

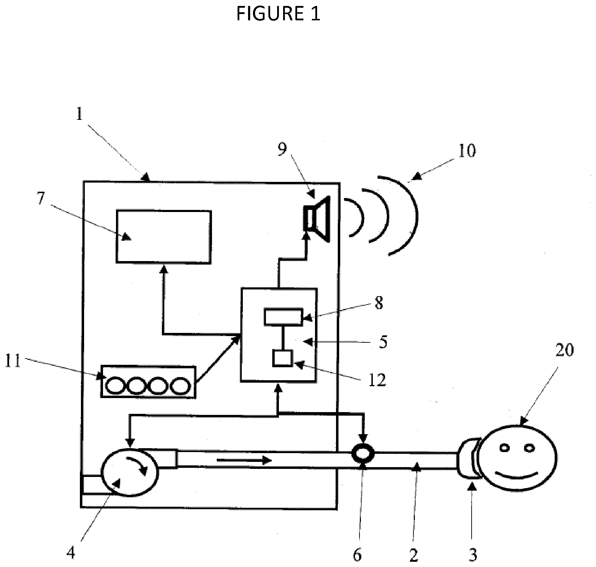

La Figure 1 schématise un mode de réalisation d'un appareil d'assistance ventilatoire ou ventilateur 1 médical selon la présente invention.

Le ventilateur 1 comprend une micro-soufflante motorisée 4 délivrant un flux de gaz d'assistance respiratoire, typiquement un flux d'air ou d'air enrichi en oxygène, dans un circuit ventilatoire 2, encore appelé circuit patient, comprenant un ou plusieurs passages ou lignes de gaz, reliant fluidiquement le ventilateur 1 aux voies aériennes d'un patient 20, par l'intermédiaire d'une interface patient 3, tel un masque respiratoire ou une sonde d'intubation.

La source de gaz 4, typiquement une micro-soufflante motorisée, encore appelée turbine ou compresseur, est pilotée par des moyens de commande pour délivrer le flux de gaz respiratoire et générer des niveaux de pression (Ph, Pb) par intermittence, pendant des durées (Dh, Db) préfixées.

La micro-soufflante motorisée 4 est équipée d'un moteur électrique animant une roue à ailettes servant à créer le flux de gaz qui est ensuite délivré en amont du circuit patient formé d'un ou plusieurs passages de gaz, tels des conduits ou analogues, servant à véhiculer le gaz jusqu'au patient 20.

La source de gaz 4 délivre le flux de gaz respiratoire et génère des niveaux de pression (Ph, Pb) pendant des durées préfixées (Dh, Db).

Généralement, la première durée (Dh) de la première phase est inférieure à 4 secondes, et/ou la deuxième durée (Db) de la deuxième phase est inférieure à 12 secondes. En outre, la durée totale de chaque cycle (Dh + Db) est inférieure à 15 secondes, typiquement inférieure à 8 secondes, typiquement de l'ordre de 5 secondes.

Pendant la première phase de première durée (Dh) de chaque cycle, le gaz est fourni à une première valeur de pression non-nulle ou « pression haute Ph », alors que pendant la deuxième phase de deuxième durée (Db), qui succède la première phase (Dh), le gaz est délivré à une seconde valeur de pression non-nulle ou « pression basse Pb », la pression haute Ph étant supérieure à la pression basse Pb, c'est-à-dire qu'on a : 0 < Pb < Ph.

De préférence, la pression haute (Ph) délivrée par la source de gaz est comprise entre 100 et 400 mm H₂O, de préférence entre 150 et 300 mm H₂O, typiquement de l'ordre de 200 mm H₂O.

De façon analogue, la pression basse (Pb) délivrée par la source de gaz est préférentiellement comprise entre 20 et 90 mm H₂O, de préférence entre 30 et 70 mm H₂O, typiquement de l'ordre de 40 mm H₂O.

Les moyens de commande pilotent alors la source de gaz de manière à délivrer le flux de gaz et à générer les niveaux de pression (Ph, Pb) pendant les durées préfixées (Dh, Db).

Selon un autre mode de réalisation (non représenté), la micro-soufflante 4 peut être remplacée par une autre source de gaz, tel qu'une vanne reliée à une canalisation de gaz, en particulier une canalisation de gaz se terminant par une prise murale agencée sur un mur d'un bâtiment hospitalier et alimentée en un gaz respiratoire, tel de l'air ou de l'oxygène, à une pression de quelques bars absolus, typiquement de l'ordre de 4 bar abs environ.

Par ailleurs, dans le ventilateur 1 de la Figure 1, on prévoit également des moyens de mesure 6 permettant de mesurer au moins un paramètre de flux représentatif du flux de gaz, typiquement la pression ou le débit de gaz insufflé par le respirateur, et de délivrer au moins un signal de flux représentatif dudit au moins un paramètre de flux mesuré.

Par exemple, le paramètre de flux de gaz est la pression du gaz dans le circuit ventilatoire 2 et les moyens de mesure 6 comprennent un capteur de pression dont la prise de pression est agencée dans ledit circuit 2 de manière à y mesurer la pression qui y règne.

Dans le mode de réalisation de la Figure 1, la prise de pression 6 est agencée hors du ventilateur. Toutefois, selon le mode de réalisation considéré, elle peut se trouver aussi bien hors que dans le ventilateur 1.

Une fois la mesure du (ou des) paramètre de flux de gaz opérée, le signal de flux correspondant, par exemple un signal de débit ou de pression, est analysé par des moyens de traitement de signal 5 qui peuvent alors y détecter des compressions thoraciques ou CT, et donc en déduire qu'un massage cardiaque est en cours de réalisation sur le patient 20, et dès lors également en déterminer la fréquence F desdites CT. De préférence, le signal est filtré comme expliqué ci-dessous.

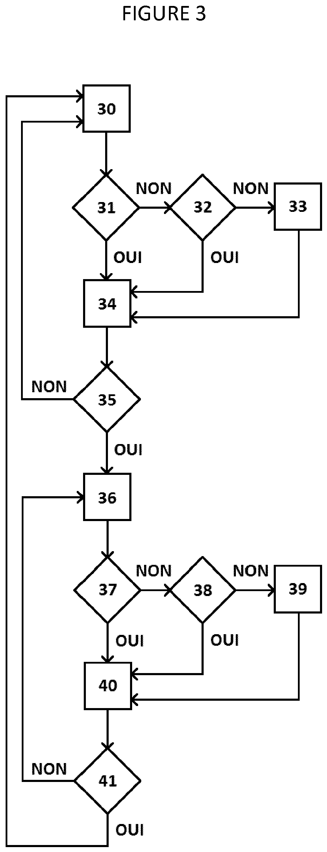

Les moyens de traitement de signal 5 comprennent par exemple un microprocesseur 8 programmable, notamment avec un algorithme de traitement, comme expliqué ci-après en rapport avec la Figure 3 notamment.

Plus précisément, les moyens de traitement de signal 5 sont aptes à et conçus pour, c'est-à-dire configurés pour comparer le signal de flux à une ou plusieurs valeurs-seuils représentatives de la réalisation d'un massage cardiaque, c'est-à-dire correspondant à des CT en cours de réalisation sur le patient 20. Ces valeurs-seuils sont enregistrées par des moyens de mémorisation 12 comprenant une mémoire de stockage, par exemple une mémoire flash. Ces valeurs-seuils peuvent être des valeurs numériques, des tableaux de valeurs, des courbes...

L'appareil comprend en outre des moyens de filtrage, par exemple une moyenne glissante calculée sur les dernières valeurs d'un signal conservées en mémoire. En effet, les compressions thoraciques sont avantageusement mises en évidence à l'aide d'un filtrage des signaux de pression et/ou de débit sous la forme d'une soustraction de moyennes glissantes. Ainsi, pour un même signal, de pression ou de débit, on peut par exemple calculer une « petite » moyenne glissante sur une durée de 10 à 100 ms, typiquement 25 ms, et une « grande » moyenne glissante sur une durée de 50 à 300 ms, typiquement 150 ms. La soustraction de la grande moyenne glissante à la petite moyenne glissante par exemple permet alors de faire apparaître les variations dudit signal, sous la forme d'un signal filtré. Les compressions thoraciques CT sont alors détectées lors du dépassement de valeurs-seuils par ce signal filtré.

En d'autres termes, selon la présente invention, le fonctionnement de l'appareil est préférentiellement basé sur une utilisation de valeurs de signal de flux filtrées, en particulier des valeurs de pression ou de débit filtrées.

Une fois détectées, les CT peuvent alors être comptabilisées par des moyens de comptabilisation inclus dans les moyens de traitement de signal 5 afin d'en déduire une valeur de fréquence de massage cardiaque, en calculant sur une certaine durée le rapport entre le nombre de compressions thoraciques et ladite durée. De préférence, ladite durée est comprise entre 1 et 15 secondes, de préférence entre 2 et 10 secondes, typiquement de l'ordre de 4 à 8 secondes, par exemple 6 secondes environ.

Une fois que la fréquence F du massage cardiaque est estimée par les moyens de traitement de signal 5, cette valeur peut être affinée en compensant les CT réelles qui sont masquées par les fortes variations de pression et de débit qui interviennent au début et à la fin des durées (Dh, Db), par l'introduction de CT virtuelles et affichage sur un écran d'affichage 7 ou analogue de l'appareil 1, d'une valeur de fréquence corrigée F_{c} prenant en compte ces CT masquées, c'est-à-dire non détectées, pour déterminer l'information de fréquence la plus appropriée à fournir au secouriste.

En d'autres termes, les moyens de traitement de signal 5 sont adaptés, c'est-à-dire configurés, pour fournir aux moyens d'affichage 7, une valeur de fréquence corrigée F_{c} correspondant à la valeur de fréquence F déterminée immédiatement avant le début d'une durée (Dh, Db) donnée correspondant à une durée pendant laquelle la micro-soufflante 4 du ventilateur 1 fournit du gaz respiratoire et génère un niveau de pression (Ph, Pb), lorsque les moyens de traitement de signal 4 ne détectent pas de compression thoracique pendant une partie de ladite durée (Dh, Db) considérée par traitement du signal de flux filtré.

Cette valeur de fréquence corrigée F_{c} est alors affichée sur l'écran d'affichage 7 du ventilateur 1, en lieu et place de la fréquence réelle qui est elle erronée du fait de l'absence de détection de certaines CT.

Ainsi, au début de chaque durée (Dh, Db), pendant un certain temps (Th, Tb) compris dans ladite durée (Dh, Db), par exemple entre 10% et 60% de ladite durée (Dh, Db), typiquement 30% (on a en particulier 0 < Th < Dh et 0 < Tb < Db), on autorise l'enregistrement par l'appareil 1 de compressions thoraciques virtuelles si aucune CT réelle n'est détectée lorsque la période correspondant à la fréquence calculée est écoulée.

Ainsi, on évite de sous-estimer la fréquence du massage cardiaque qui serait due à une absence de détection.

En outre, si une CT virtuelle est enregistrée, on empêche la détection d'une nouvelle CT réelle pendant une durée prédéfinie, par exemple entre 50 et 350 ms, typiquement 120 ms, pour ne pas comptabiliser deux fois cette compression thoracique sous la forme d'une CT virtuelle d'une part, et d'une CT réelle, d'autre part. En effet, lorsque le massage cardiaque est manuel notamment, la fréquence du massage n'est pas d'une régularité parfaite, et une CT réelle peut intervenir légèrement plus tard que la fréquence calculée le laisse prévoir. Si tel est le cas, et que cette CT réelle a déjà été prise en compte par le biais d'une CT virtuelle, il ne faut plus tenir compte de la CT réelle, pour ne pas surestimer la fréquence du massage.

L'appareil 1 selon la présente invention permet d'assister le secouriste en lui indiquant une information de fréquence, par exemple la valeur numérique de la fréquence, ou des indications de type « trop rapide », « trop lent »..., ce qui lui donnera des informations très utiles quant à l'efficacité du massage cardiaque qu'il est en train de pratiquer. Ces informations de fréquence sont affichées grâce à des moyens d'affichage 7, tel un écran ou un afficheur.

Par ailleurs, des moyens de mesure de durée, par exemple un chronomètre intégré à un processeur 8, peuvent également être prévus pour mesurer la durée pendant laquelle le massage cardiaque est réalisé. Cette durée sera ensuite transmise aux moyens d'affichage 7 pour afficher cette durée de massage cardiaque à destination du personnel soignant et ainsi lui apporter instantanément une autre information importante.

Le ventilateur 1 peut comprendre également une fonction de type « métronome » permettant d'assister le personnel soignant dans la réalisation du massage cardiaque selon un rythme donné.

Le ventilateur 1 comporte en outre une coque ou capotage externe rigide, par exemple en polymère, incorporant tout ou partie des moyens et éléments susmentionnés, en particulier la micro-soufflante 4, les moyens de traitement de signal 5, y compris le (ou les) microprocesseur, les moyens de mémorisation, les moyens d'affichage 7, au moins une partie du circuit ventilatoire 2...

Le ventilateur 1 est alimenté en courant électrique issu d'une ou plusieurs batteries, rechargeables ou non, de l'alimentation électrique du véhicule de secours qu'il équipe ou du secteur, donc sous une tension pouvant aller jusqu'à environ 230 V.

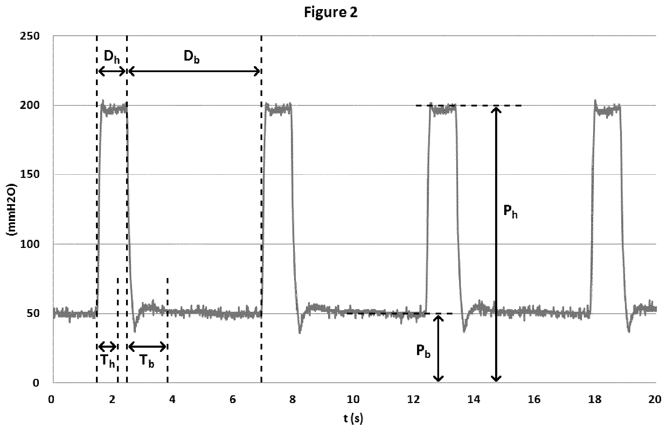

La Figure 2 est un exemple d'un enregistrement d'un paramètre de pression mesuré en fonction du temps, en l'absence de massage cardiaque. Cet enregistrement a été réalisé avec un appareil ventilatoire Monnal T60 commercialisé par la société Air Liquide Médical Systems.

La Figure 2 schématise les durées Dh et Db pendant lesquelles sont maintenus les niveaux respectifs de pression de gaz Ph et Pb, ainsi que les temps Th et Tb respectivement compris dans lesdites durées Dh et Db, pendant lesquels on autorise l'enregistrement par l'appareil 1 de compressions thoraciques virtuelles si aucune CT réelle n'est détectée lorsque la période correspondant à la fréquence calculée est écoulée.

Le logigramme de la Figure 3 illustre le fonctionnement d'un algorithme pouvant être mis en oeuvre au sein des moyens de traitement de signal 5 du ventilateur 1 selon l'invention.

Comme on le voit, le ventilateur 1 commence d'abord par appliquer le niveau de pression Ph (en 30). Si le temps écoulé depuis le début du niveau de pression Ph est inférieur au temps Th (en 31), le ventilateur 1 vérifie si des CT réelles sont détectées (en 32). Si c'est le cas, ces CT sont directement enregistrées pour le calcul de la fréquence (en 34). Sinon des CT virtuelles sont ajoutées (en 33) pour être prises en compte dans le calcul de la fréquence (en 34) et compenser les CT réelles masquées par la ventilation mécanique. Si le temps écoulé depuis le début du niveau de pression Ph est supérieur au temps Th (en 31), seules les CT réelles sont enregistrées pour le calcul de la fréquence (en 34).

Si le temps écoulé depuis le début du niveau de pression Ph est inférieur à la durée Dh (en 35), le ventilateur 1 continue d'appliquer le niveau de pression Ph (en 30). Sinon le ventilateur applique le niveau de pression Pb (en 36). Si le temps écoulé depuis le début du niveau de pression Pb est inférieur au temps Tb (en37), le ventilateur 1 vérifie si des CT réelles sont détectées (en 38). Si c'est le cas, ces CT sont directement enregistrées pour le calcul de la fréquence (en 40). Sinon des CT virtuelles sont ajoutées (en 39) pour être prises en compte dans le calcul de la fréquence (en 40) et compenser les CT réelles masquées par la ventilation mécanique. Si le temps écoulé depuis le début du niveau de pression Pb est supérieur au temps Tb (en 37), seules les CT réelles sont enregistrées pour le calcul de la fréquence (en 40).

Si le temps écoulé depuis le début du niveau de pression Pb est inférieur à la durée Db (en 41), le ventilateur 1 continue d'appliquer le niveau de pression Pb (en 36). Sinon le ventilateur applique le niveau de pression Ph (en 30), et ainsi de suite de façon cyclique.

Dit autrement, l'observation permanente des signaux de pression, voire de débit, permet par une analyse du signal classique, par exemple par filtrage ou reconnaissance de motif, de détecter le massage cardiaque. Une fois le massage détecté, la fréquence peut être calculée par les mêmes techniques usuelles de traitement du signal.

La durée depuis le début du massage peut aussi être affichée. Ainsi, elle peut être chronométrée par le processeur.

L'appareil d'assistance respiratoire selon l'invention peut être de type portatif et muni d'une poignée ou analogue de transport.

A des fins de mobilité, ladite au moins une source de gaz peut être une turbine et/ou une bouteille de gaz sous pression, typiquement munie d'un détendeur.

L'appareil d'assistance respiratoire selon l'invention est particulièrement bien adapté à une utilisation par un secouriste, tel un médecin du SAMU, un pompier, un infirmier ou analogue, pour ventiler une personne, c'est-à-dire un patient, en arrêt cardio-pulmonaire, pendant qu'elle est soumise à des compressions thoraciques (CT) dans le cadre d'un massage cardiaque opéré par ledit secouriste.

## Revendications

1. Appareil d'assistance respiratoire (1) comprenant :
- une source de gaz (4) apte à délivrer un flux de gaz respiratoire dans un circuit ventilatoire (2) destiné à être relié à un patient (20), le flux de gaz respiratoire étant délivré par ladite source de gaz (4) de manière à générer et à maintenir plusieurs niveaux de pression de gaz (Ph, Pb), pendant des durées (Dh, Db) préfixées,
- des moyens de mesure (6) aptes à et conçus pour mesurer au moins un paramètre de flux représentatif du flux de gaz choisi parmi la pression du gaz et le débit de gaz, et pour délivrer au moins un signal de flux représentatif dudit paramètre de flux,
- des moyens de traitement de signal (5) conçus pour et aptes à :
i) analyser et déduire dudit signal de flux, une existence ou une absence de compressions thoraciques (CT) pratiquées sur ledit patient,
ii) déterminer la valeur de fréquence (F) desdites compressions thoraciques (CT) en cas d'existence de compressions thoraciques(CT),
iii) fournir ladite valeur de fréquence (F) à des moyens d'affichage (7),
- et des moyens d'affichage (7) permettant d'afficher des informations,
**caractérisé en ce que** les moyens de traitement de signal sont adaptés pour fournir aux moyens d'affichage, une valeur de fréquence corrigée (F_{c}) correspondant à la valeur de fréquence (F) déterminée immédiatement avant le début d'une durée (Dh, Db) considérée, lorsque les moyens de traitement de signal ne détectent pas de compression thoracique (CT) pendant une partie de ladite durée (Dh, Db) considérée.

2. Appareil selon la revendication précédente, **caractérisé en ce que** les moyens d'affichage (7) sont configurés pour afficher la valeur de fréquence corrigée (F_{c}), pendant au moins une partie de la durée (Dh, Db) considérée.

3. Appareil selon l'une des revendications précédentes, **caractérisé en ce que** les moyens d'affichage (7) sont configurés pour afficher, directement ou indirectement, la fréquence sous la forme d'une valeur numérique, d'une représentation graphique, d'une indication ou de tout symbole illustratif.

4. Appareil selon l'une des revendications précédentes, **caractérisé en ce que** la source de gaz (4) délivre le flux de gaz respiratoire et maintient les niveaux de pression de gaz (Ph, Pb) dudit flux, pendant des durées préfixées (Dh, Db), de sorte que :
- le gaz est maintenu à pression haute (Ph) pendant une première durée (Dh), et
- le gaz est maintenu à pression basse (Pb) pendant une deuxième durée (Db), avec :
0 < Pb < Ph, la deuxième durée (Db) succédant à la première durée (Dh),
de préférence les durées (Dh, Db) sont alternées de façon cyclique.

5. Appareil selon l'une des revendications précédentes, **caractérisé en ce qu'**il comprend des moyens de commande pilotant la source de gaz (4) de manière à délivrer le flux de gaz et à générer les niveaux de pression (Ph, Pb) pendant les durées préfixées (Dh, Db).

6. Appareil selon l'une des revendications précédentes, **caractérisé en ce que** la source de gaz (4) est une micro-soufflante motorisée.

7. Appareil selon l'une des revendications précédentes, **caractérisé en ce que** les moyens de mesure (6) comprennent un capteur de débit ou un capteur de pression.

8. Appareil selon l'une des revendications précédentes, **caractérisé en ce que** les moyens de traitement de signal (5) comprennent au moins un microprocesseur (8) mettant en oeuvre au moins un algorithme.

9. Appareil selon l'une des revendications précédentes, **caractérisé en ce que** les moyens d'affichage (7) comprennent un écran d'affichage.

10. Appareil selon l'une des revendications précédentes, **caractérisé en ce qu'**il comporte des moyens de filtrage configurés pour filtrer le signal de flux représentatif du paramètre de flux provenant des moyens de mesure.

11. Appareil selon l'une des revendications précédentes, **caractérisé en ce qu'**il comporte des moyens de mémorisation (12) conçus pour mémoriser des données.

12. Appareil selon l'une des revendications précédentes, **caractérisé en ce que** les moyens de traitement de signal (5) sont configurés pour déterminer la valeur de fréquence (F) des compressions thoraciques (CT) en calculant sur une durée donnée, le rapport entre le nombre de compressions thoraciques (CT) et ladite durée donnée, de préférence une durée comprise entre 1 et 15 secondes.

13. Appareil selon l'une des revendications précédentes, **caractérisé en ce qu'**il comporte des moyens de comptabilisation des compressions thoraciques (CT) détectées pendant une période de temps donnée.

14. Appareil selon l'une des revendications précédentes, **caractérisé en ce que** les moyens de traitement de signal (5) sont configurés pour fournir aux moyens d'affichage (7), la valeur de fréquence corrigée (F_{c}), lorsque les moyens de traitement de signal (5) ne détectent pas de compression thoracique (CT) pendant un temps (Th, Tb) égal à 10 à 60% de la durée (Dh, Db) considérée.

## Patentansprüche

1. Einrichtung zur Atmungsunterstützung (1), umfassend:
- eine Gasquelle (4), die in der Lage ist, eine Strömung von Atemgas in einen Beatmungskreislauf (2) zu liefern, der dazu bestimmt ist, mit einem Patienten (20) verbunden zu werden, wobei die Strömung von Atemgas von der Gasquelle (4) geliefert wird, um mehrere Gasdruckniveaus (Ph, Pb) für vorbestimmte Dauern (Dh, Db) zu erzeugen und aufrechtzuerhalten,
- Messmittel (6), die in der Lage und ausgelegt sind, mindestens einen Strömungsparameter zu messen, der für die Gasströmung repräsentativ ist, ausgewählt aus dem Gasdruck und dem Gasdurchsatz, und mindestens ein Strömungssignal zu liefern, das für den Strömungsparameter repräsentativ ist,
- Signalverarbeitungsmittel (5), die ausgelegt sind zum und in der Lage sind zum:
i) Analysieren und Ableiten aus dem Strömungssignal, eines Vorhandenseins oder Fehlens von Thoraxkompressionen (CT), die an dem Patienten durchgeführt werden,
ii) Bestimmen des Frequenzwertes (F) der Thoraxkompressionen (CT) im Falle des Vorhandenseins von Thoraxkompressionen (CT),
iii) Bereitstellen des Frequenzwertes (F) an die Anzeigemittel (7),
- und Anzeigemittel (7), um Anzeigen von Informationen zu ermöglichen,
**dadurch gekennzeichnet, dass** die Signalverarbeitungsmittel angepasst sind, um den Anzeigemitteln einen korrigierten Frequenzwert (F_{c}) bereitzustellen, der dem Frequenzwert (F) entspricht, der unmittelbar vor Beginn einer betrachteten Dauer (Dh, Db) bestimmt wird, wenn die Signalverarbeitungsmittel keine Thoraxkompression (CT) während eines Teils der betrachteten Dauer (Dh, Db) detektieren.

2. Einrichtung nach dem vorstehenden Anspruch, **dadurch gekennzeichnet, dass** die Anzeigemittel (7) konfiguriert sind, um den korrigierten Frequenzwert (F_{c}) während mindestens eines Teils der betrachteten Dauer (Dh, Db) anzuzeigen.

3. Einrichtung nach einem der vorstehenden Ansprüche, **dadurch gekennzeichnet, dass** die Anzeigemittel (7) konfiguriert sind, um die Frequenz direkt oder indirekt in Form eines Zahlenwertes, einer grafischen Darstellung, eines Hinweises oder eines beliebigen illustrativen Symbols anzuzeigen.

4. Einrichtung nach einem der vorstehenden Ansprüche, **dadurch gekennzeichnet, dass** die Gasquelle (4) die Strömung von Atemgas liefert und die Gasdruckniveaus (Ph, Pb) der Strömung während vorbestimmten Dauern (Dh, Db) aufrechterhält, sodass:
- das Gas während einer ersten Dauer (Dh) auf hohem Druck (Ph) gehalten wird,
und
- das Gas während einer zweiten Dauer (Db) auf niedrigem Druck (Pb) gehalten wird, mit:
0 < Pb < Ph, wobei die zweite Dauer (Db) auf die erste Dauer (Dh) folgt, die Dauern (Dh, Db) vorzugsweise zyklisch gewechselt werden.

5. Einrichtung nach einem der vorstehenden Ansprüche, **dadurch gekennzeichnet, dass** sie Steuermittel umfasst, welche die Gasquelle (4) ansteuern, um die Gasströmung zu liefern und die Druckniveaus (Ph, Pb) während der vorbestimmten Dauern (Dh, Db) zu erzeugen.

6. Einrichtung nach einem der vorstehenden Ansprüche, **dadurch gekennzeichnet, dass** die Gasquelle (4) ein angetriebenes Mikrogebläse ist.

7. Einrichtung nach einem der vorstehenden Ansprüche, **dadurch gekennzeichnet, dass** die Messmittel (6) einen Durchsatzsensor oder einen Drucksensor umfassen.

8. Einrichtung nach einem der vorstehenden Ansprüche, **dadurch gekennzeichnet, dass** die Signalverarbeitungsmittel (5) mindestens einen Mikroprozessor (8) umfassen, der mindestens einen Algorithmus ausführt.

9. Einrichtung nach einem der vorstehenden Ansprüche, **dadurch gekennzeichnet, dass** die Anzeigemittel (7) einen Anzeigebildschirm umfassen.

10. Einrichtung nach einem der vorstehenden Ansprüche, **dadurch gekennzeichnet, dass** sie Filtermittel umfasst, die konfiguriert sind, um das für den Strömungsparameter repräsentative Strömungssignal aus den Messmitteln zu filtern.

11. Einrichtung nach einem der vorstehenden Ansprüche, **dadurch gekennzeichnet, dass** sie Speichermittel (12) beinhaltet, die zum Speichern von Daten ausgelegt sind.

12. Einrichtung nach einem der vorstehenden Ansprüche, **dadurch gekennzeichnet, dass** die Signalverarbeitungsmittel (5) konfiguriert sind, um den Frequenzwert (F) von Thoraxkompressionen (CT) zu bestimmen, indem sie über eine gegebene Dauer das Verhältnis zwischen der Anzahl der Thoraxkompressionen (CT) und der gegebenen Dauer, vorzugsweise eine Dauer zwischen 1 und 15 Sekunden, berechnen.

13. Einrichtung nach einem der vorstehenden Ansprüche, **dadurch gekennzeichnet, dass** sie Mittel zum Zählen der während einer gegebenen Zeitdauer detektierten Thoraxkompressionen (CT) beinhaltet.

14. Einrichtung nach einem der vorstehenden Ansprüche, **dadurch gekennzeichnet, dass** die Signalverarbeitungsmittel (5) konfiguriert sind, um den Anzeigemitteln (7) den korrigierten Frequenzwert (F_{c}) bereitzustellen, wenn die Signalverarbeitungsmittel (5) keine Thoraxkompression (CT) für eine Zeit (Th, Tb) gleich 10 bis 60 % der betrachteten Dauer (Dh, Db) detektieren.

## Claims

1. Respiratory assistance apparatus (1) comprising:
- a source of gas (4) able to deliver a flow of respiratory gas in a ventilatory circuit (2) intended to be connected to a patient (20), the flow of respiratory gas being delivered by said source of gas (4) in such a way as to generate and maintain several levels of gas pressure (Ph, Pb), during preset durations (Dh, Db),
- measurement means (6) able and designed to measure at least one flow parameter representative of the flow of gas chosen from the pressure of the gas and the gas flow rate, and to deliver at least one flow signal representative of said flow parameter,
- signal processing means (5) designed and able to:
i) analyse and deduce from said flow signal, an existence or an absence of chest compressions (CT) practiced on said patient,
ii) determine the frequency value (F) of said chest compressions (CT) in case of the existence of chest compressions (CT),
iii) provide said frequency value (F) to display means (7),
- and display means (7) that make it possible to display information,
**characterised in that** the signal processing means are suitable for providing to the display means a corrected frequency value (F_{c}) corresponding to the frequency value (F) determined immediately before the start of a duration (Dh, Db) considered, when the signal processing means do not detect any chest compression (CT) during a portion of said duration (Dh, Db) considered.

2. Apparatus according to the preceding claim, **characterised in that** the display means (7) are configured to display the corrected frequency value (F_{c}), for at least one portion of the duration (Dh, Db) considered.

3. Apparatus according to one of the preceding claims, **characterised in that** the means for displaying (7) are configured to display, directly or indirectly, the frequency in the form of a numerical value, of a graphical representation, of an indication or of any illustrative symbol.

4. Apparatus according to one of the preceding claims, **characterised in that** the source of gas (4) delivers the flow of respiratory gas and maintains the levels of gas pressure (Ph, Pb) of said flow, during preset durations (Dh, Db), in such a way that:
- the gas is maintained at high pressure (Ph) during a first duration (Dh), and
- the gas is maintained at low pressure (Pb) for a second duration (Db), with:
0 < Pb < Ph, with the second duration (Db) succeeding the first duration (Dh), more preferably the durations (Dh, Db) are alternated cyclically.

5. Apparatus according to one of the preceding claims, **characterised in that** it comprises means for controlling the source of gas (4) in such a way as to deliver the flow of gas and to generate the levels of pressure (Ph, Pb) during preset durations (Dh, Db).

6. Apparatus according to one of the preceding claims, **characterised in that** the source of gas (4) is a motorised micro-fan.

7. Apparatus according to one of the preceding claims, **characterised in that** the means for measurement (6) comprise a flow rate sensor or a pressure sensor.

8. Apparatus according to one of the preceding claims, **characterised in that** the signal processing means (5) comprise at least one microprocessor (8) that implements at least one algorithm.

9. Apparatus according to one of the preceding claims, **characterised in that** the display means (7) comprise a display screen.

10. Apparatus according to one of the preceding claims, **characterised in that** it comprises filtering means configured to filter the flow signal representative of the flow parameter coming from the measurement means.

11. Apparatus according to one of the preceding claims, **characterised in that** it comprises means for memorising (12) designed to memorise data.

12. Apparatus according to one of the preceding claims, **characterised in that** the signal processing means (5) are configured to determine the frequency value (F) of the chest compressions (CT) by calculating over a given duration, the ratio between the number of chest compressions (CT) and said given duration, more preferably a duration between 1 and 15 seconds.

13. Apparatus according to one of the preceding claims, **characterised in that** it comprises means for counting chest compressions (CT) detected during a given period of time.

14. Apparatus according to one of the preceding claims, **characterised in that** the signal processing means (5) are configured to provide the display means (7) with the corrected frequency value (F_{c}), when the signal processing means (5) do not detect any chest compression (CT) for a time (Th, Tb) equal to 10 to 60% of the duration (Dh, Db) considered.
